# EUROPEAN PATENT APPLICATION

(11) **EP 4 245 268 A1**
(43) Date of publication of application: **20.09.2023**
(21) Application number: 20961466.8
(22) Date of filing: 12.11.2020
(51) Int. Cl.: A61F 2/44, A61F 5/04

(54) **IMPLANT FOR THE INTERVERTEBRAL DISC SPACE FOR TREATING SCOLIOSIS, KYPHOSIS, STENOSIS AND FRACTURES OF THE SPINAL COLUMN**

(71) Applicant: Burgos Flores, Jesús, 28043 Madrid (ES)
(72) Inventor: Burgos Flores, Jesús, 28043 Madrid (ES)
(74) Representative: Urizar Anasagasti, Jesus Maria
(86) International application number: PCT/ES2020/070704
(87) International publication number: WO 2022/101524

(57) **Abstract**

Disclosed is an implant for the intervertebral disc space, for the treatment of scoliosis, kyphosis, stenosis and fractures of the spinal column, which comprises a substantially cylindrical, inflatable, flexible, biocompatible element that has a morphology dependent on the problem to be treated and is suitable for insertion into the intervertebral disc space. The implant can be inflated using an external pressure system in order to allow the injured disc to be lifted, and it can be filled with a material (2) which, once solidified, has an elastic hardness similar to that of a healthy intervertebral disc at the vertebral level in which the implant is to be inserted, said elastic hardness being higher in the area of the lumbar vertebrae and lower in the dorsal or cervical area.

## Description

### Technical field

The sector to which the invention belongs is that of surgical instruments, devices or procedures for the treatment of bones or joints.

The invention relates to an implant for the intervertebral disc space for the treatment of deformities and other disorders of the spinal column, consisting of an inflatable balloon of different shapes and capable of adapting to the disc space when inflated. This balloon is filled with materials that have an elastic hardness that turns out to be similar to that of a healthy intervertebral disc at the vertebral level in which the implants are inserted. With its implementation, the intervertebral disc is lifted and the corresponding vertebral segment is stabilized, with consequent decompression of the medullary canal and foramina, recovering normal vertebral mobility. The field of application of the use of this implant is in the treatment of scoliosis, kyphosis, stenosis and fractures of the spinal column.

### State of the art

Between every two vertebrae is an intervertebral disc, which is the mobile elastic element that has the function of transmitting, distributing and absorbing weight loads produced by gravity and movement, thus preventing high mechanical stresses on limited areas of the vertebrae that could cause fractures or vertebral overloading with negative and irreversible repercussions.

The intervertebral disc has three components:
1. The cartilaginous endplate, which remains loosely attached to the bone vertebral plate, forming a barrier that prevents the passage of the nucleus pulposus into the vertebral body.
2. The annulus fibrosus, which is a thick fibrous band that surrounds the nucleus pulposus and is made up of several layers of collagen fibers.
3. The nucleus pulposus, which is surrounded by the endplates and the annulus fibrosus and is semi-gelatinous in nature in order to distribute the vertebral hydraulic loads in all directions, preventing the concentration of forces on limited areas.

The degeneration of the intervertebral disc at any level of the cervical, thoracic and lumbar spinal column leads to a decrease in the stress in the intervertebral disc space of the nucleus pulposus and consequently the loss of disc height with bulging of the soft portions surrounding it, in other words, the disc annulus fibrosus and the adjacent vertebral ligaments. This decrease in disc height also affects the posterior facet joints of the two vertebrae adjacent to the disc, which lose their congruence with subluxations. The bulging of the peripheral elements of the disc and the facet subluxation inserting the faceted ends into the foramina compress the nerve structures, the spinal cord and the nerve roots, which are in contact with the bulging intervertebral disc.

To date, in order to decompress the nerves and the spinal cord in the spinal column, stabilize the unstable spinal column and restore vertebral mobility, surgeons use extensive surgical approaches that require sectioning anatomical structures, opening the abdominal and/or thoracic cavity on many occasions and handling nerve and vascular structures with a high number of complications for patients, especially older ones.

Documents US2020113705A1 and US2013131808A1 disclose respective balloon-shaped implants, capable of inflating by using an external pressure system and being able to be filled with elastic materials. Both balloons are intended to replace degenerated intervertebral discs and, more specifically, the nucleus pulposus of an intervertebral disc with a percutaneous implantable nuclear prosthesis.

Document WO2009117459A2 describes a device for restoring the height of an intervertebral disc, said device including a body that is inserted into the intervertebral space, which defines top and bottom surfaces that engage in the space between two consecutive vertebrae.

Furthermore, the referenced implants do not allow their use in the treatment of scoliosis, kyphosis, stenosis or fractures of the spinal column, since they do not have suitable morphology or the structure for it, nor does the filling material offer the hardness and elasticity required for the different areas of the vertebrae in which these balloons are implanted.

No description of the use of balloons of this type for the treatment of scoliosis, kyphosis, stenosis or fractures of the spinal column is known in the medical literature either.

### Explanation of the invention

In order to achieve the proposed objectives mentioned in the previous section, the invention proposes an implant for the intervertebral disc space for the treatment of scoliosis, kyphosis, stenosis or fractures of the spinal column, which has the features of claim 1. More specifically, and as indicated in the previous point, this implant consists of a substantially cylindrical, inflatable, flexible, biocompatible balloon that is suitable for insertion into the intervertebral disc space and when inflated by an external pressure system, it allows the injured intervertebral disc to be lifted and the corresponding vertebral segment to be stabilized, while decompressing the medullary canal and foramina restores vertebral mobility. The main feature of the implant of the invention is that said balloon has, both around the perimeter and on its bases, a morphology dependent on the problem to be treated; and therein it has a filling material which, once solidified, has an elastic hardness similar to that of the healthy intervertebral disc at the vertebral level in which the implant is to be inserted.

One of the great advantages of using this type of balloons in the intervertebral disc space is that they are inserted percutaneously, using trocars that are placed through the skin in the intervertebral disc. Their insertion through the skin causes small surgical wounds that facilitate the rapid recovery of the patient and hospital discharge a few hours after the intervention.

With the present methodology, a disc can be lifted by inflating the balloon once it is inserted inside the disc and in this way the original disc height will be recovered, eliminating the bulging of the peripheral soft portions of the disc. This disc lifting also allows the congruence of the posterior facet joints to be recovered. In short, the decompression of the nerve elements: the spinal cord and the nerve roots, is achieved. Vertebral stabilization is also achieved through "annulotaxis and ligamentotaxis" that is achieved with the lifting of the intervertebral disc, while preventing abnormal movements of that vertebral disc segment. This disc lifting tightens the peripheral ligamentous elements of the disc, the annulus fibrosus, and the adjacent vertebral ligaments at the level of the affected disc, thus achieving stability of this vertebral segment. Likewise, focal kyphosis caused by the decrease in disc height is corrected and muscle functionality is improved by increasing its lever arm, especially in cases where several levels are treated.

With the present proposal for using this type of implant, vertebral imbalances caused by lesions of the disc or discs of the spinal column are corrected, regardless of the cause that leads to the loss of disc height, giving rise to kyphosis at this vertebral level, and this correction has very important consequences for the patient because it allows them to recover verticality and improve functionality.

The present therapeutic proposal also achieves spectacular results in patients with multiple discopathies that force them to have very significant muscle overloads in order to remain upright and maintain physiological verticality. This situation of vertebral imbalance causes easy fatigue and pain, especially in the elderly with significant functional limitations, who are the ones most frequently affected.

With disc lifting and maintenance of the disc height that is achieved with this elastic implant, it is possible to recover the congruence of the posterior facet joints and physiological vertebral mobility. Furthermore, this elastic element prevents fractures of the adjacent vertebrae that occur in a high percentage of cases, as a result of the use of bone cement that is currently implanted in fractured vertebrae. This complication is even more frequent in cases of discoplasty where the cement is inserted into the intervertebral disc, and this bone cement for the intervertebral disc space frequently sinks into the fragile adjacent vertebral bone (subsidence). Although performing vertebroplasties in the vertebrae adjacent to the treated vertebra has been recommended to avoid this complication, in other words, also inserting bone cement into the adjacent vertebrae "to reinforce them", this methodology does not prevent subsidence of the cement in the vertebra adjacent to the discoplasties, since the bone cement placed inside the intervertebral disc fractures the vertebral plate that is next to the cement and even more frequently in cases with osteoporosis, which is frequent in older patients.

Subsidence of the cement in the vertebral bodies adjacent to the discoplasty or the appearance of secondary vertebral fractures to the use of cement causes the loss of the correction achieved and the reappearance of the preoperative situation or even worsens it. This complication, almost inevitable with the use of bone cement in the spinal column, is avoided with the application of elastic material in the discs with a higher elasticity than the adjacent vertebrae. The use of elastic material will shorten surgery time and the number of levels to be treated, since it will not be necessary to perform adjacent vertebroplasties to prevent the appearance of fractures in the neighboring vertebrae.

### Description of the drawings

As a complement to the present description, and for the purpose of helping to make the features of the invention more readily understandable, said description is accompanied by a set of drawings which, by way of illustration and not limitation, represent the following:
Figure 1 is a side cross-section of the implant of the invention.
Figures 2 and 3 show sagittal and axial sections, respectively, in an implant with the balloon (1) located and inflated in the site corresponding to the old nucleus pulposus.
Figure 4 represents an axial cross-section of a lumbar disc from a patient with scoliosis, as shown in Figure 5.
Figures 5 and 6 show an area of the spinal column with thoraco-lumbar degenerative scoliosis, respectively before and after placing the implants of the invention.
Figure 7 shows an axial cross-section of an implant placed in a case of kyphosis with anterior vertebral imbalance or spondylolisthesis.
Figure 8 shows a side view of a case of lumbar spinal stenosis following treatment with disc implantation of the device herein proposed.
Figure 9 shows an implant placed in a case of a severe fracture of the L1 (Kummel) vertebra, without affecting the T12 endplate.
Figure 10 shows an implant placed in a case of severe fracture of the L1 (Kummel) vertebra, with an associated fracture of the T12 caudal endplate.

### Embodiment of the invention

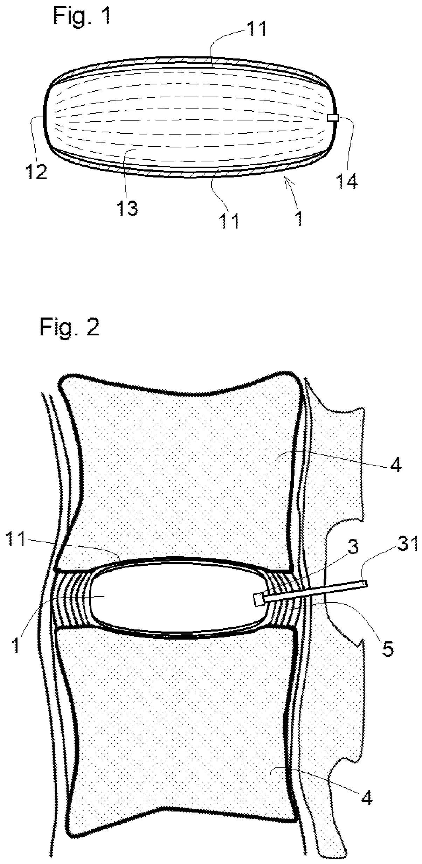

The implant for the intervertebral disc space of the present invention, intended for the recovery and treatment of scoliosis, kyphosis, stenosis or fractures of the spinal column, as can be seen in Figures 1-3, comprises a substantially cylindrical, flexible, biocompatible balloon (1) that is suitable for insertion into the intervertebral disc space, which is provided with a connection (3) through which it can be connected with a tube (31) that allows fluid to be inserted with the help of an external pressure system, thus achieving its stabilization in the disc and disc lifting, and consequently the decompression of the injured neurological elements.

According to the invention, in this balloon (1) at least one of its bases (11) is rigid or semi-rigid and both bases (11) have a perimeter morphology dependent on the problem to be treated. For its part, the perimeter envelope (12) is flexible and its height is determined by the separation that must exist between intervertebral discs, once said balloon is inflated. In this way, the balloon (1) can be adapted to the recovery and treatment of the aforementioned conditions.

According to another important feature of the invention, the inside of said balloon (1) is filled with a material (2) which, once solidified, has an elastic hardness similar to that of the healthy intervertebral disc at the vertebral level in which the implant is to be inserted. In general, the elastic hardness of the filling material (2) of the balloon (1) is comprised between Shore 50A and Shore 90A.

In a preferential embodiment, the balloon (1) has radiopaque markers (17) on both bases that allow the exact location of said balloon to be observed when it is placed between two vertebrae (4).

The material (2) that fills the balloon (1) can be any type of elastic material that is injected in the form of liquid or gel, and solidifies once injected inside the balloon (1), acquiring the elastic hardness similar to that of the healthy intervertebral disc at the vertebral level in which the implant is to be inserted.

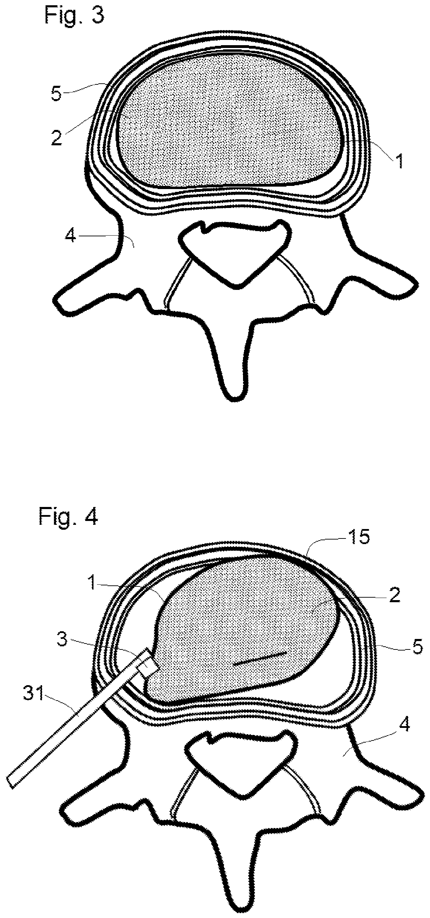

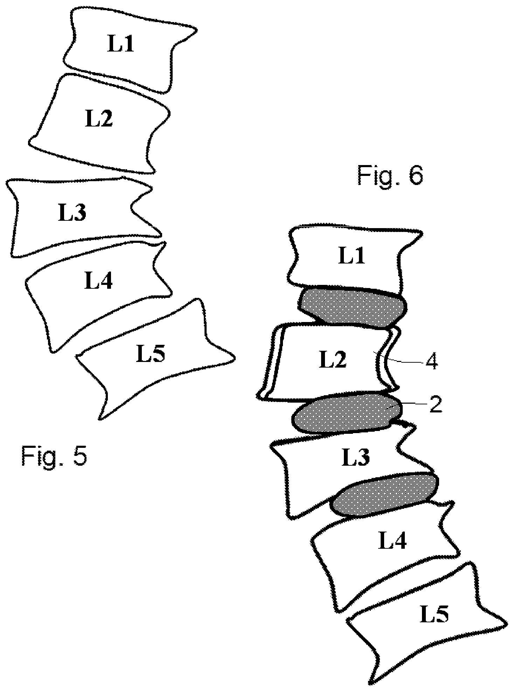

Examples of the morphology and structure of these balloons (1) for various treatments are described below. Starting with vertebral deformations, such as degenerative scoliosis, for example, the one observed in the patient in Figure 5. In this case, an implant like the one represented in Figure 4 is inserted in an axial section of a lumbar disc, where it is shown how the morphology of the implant has the largest contact surface in the anterior part (15) and in the concavity of scoliosis, which is precisely where the greatest corrective forces for correcting the deformity should be applied and where the implant will suffer greater mechanical strain.

The medical literature in this pathology contains no descriptions of percutaneous surgical treatment with the methodology presented herein. There is also no knowledge of patents related to the percutaneous treatment of this pathology, nor to the methodology and type of implant herein proposed, performing it openly.

The insertion of the trocar into the disc (5) is preferably performed from the side of convexity, where it is technically very easy to reach the disc and properly place the implant in the anterior situation and in the concavity of scoliosis. Taking into account that this pathology frequently affects elderly patients and that the degenerative disc changes are very severe, with an almost total absence of tissue in the intervertebral disc space and frequent sclerosis of the endplates, the implant is adapted to these characteristics. In the design of this implant, the importance of applying corrective forces for correcting scoliosis at the appropriate level, which is the concavity of scoliosis and the anterior part of the vertebral body, has also been taken into account to correct kyphosis. The implant is made in such a way that these two levels will be where the contact surfaces of the implant with the vertebral bodies will be more extensive.

The implant, i.e., the shaped balloon, maintains the indurated cephalic and caudal surfaces to maintain contact with the generally sclerotic vertebral endplates. The implant is filled with cement having elastic characteristics as described previously, taking into account the frequent associated sclerosis of the endplates.

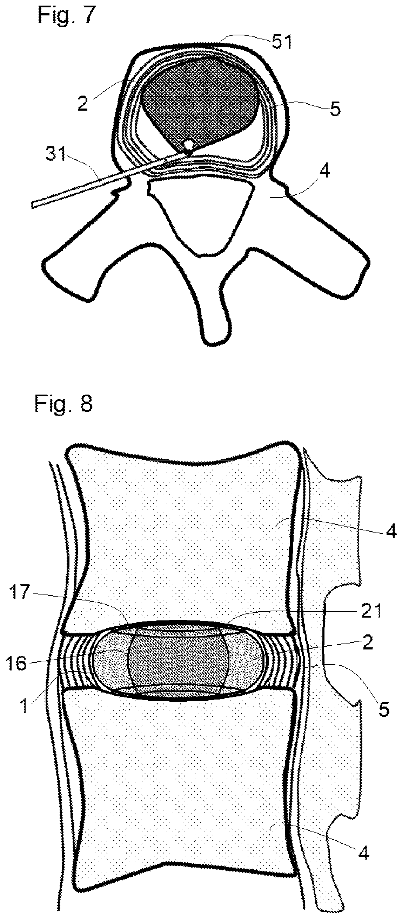

For the treatment of kyphosis, anterior vertebral imbalances and spondylolisthesis, the same implant described for the treatment of degenerative scoliosis is used, but positioned differently. The area where the maximum corrective forces will be applied and where the greatest mechanical stresses will be maintained after correction is in the anterior part of the vertebral body and this anterior and central area is where the implant must be placed (see Figure 7). For this, the percutaneous entry point in the disc must be made subsequently by using a trocar that allows 60-90° angulation of the distal end with external control, to allow the implant to be positioned in the anterior and central part of the vertebral body and said position is where the implant must be inflated.

In the treatment of disc degeneration, whether in the cervical, thoracic or lumbar spinal column, and lumbar spinal stenosis, the implant shown in Figure 8 is used. In this case, the implant is made up of a balloon (1) made of a flexible, biocompatible material that is capable of being inflated, and which can be inserted into the intervertebral disc by a trocar. When this balloon (1) is inflated, it increases in size inside the disc, taking on a substantially cylindrical shape. This central cylinder is in contact with and surrounded by a second balloon (16), which can also be inflated around the first cylinder (1) and independently of it.

When this implant is used, in cases of disc degeneration or lumbar spinal stenosis, with the aim of increasing the stability of the implant and providing greater disc mobility, causing a smoother transition to the annulus fibrosus, the central cylinder (16) is filled with an elastic cement (21) having elasticity less than or equal to the cement (2) that fills the peripheral cylinder (1).

The balloon (1) has radiopaque circular markers (17) on both bases that allow the exact location of the implants relative to the vertebral endplates to be observed, said endplates being where the balloon must be located to facilitate its load-transmitting function. These markers can be viewed when placing the balloon in the intervertebral disc space under radioscopic control, navigation, or when placed robotically.

Fractures of the vertebral body are associated with sinking of the endplates that causes an alteration of the normal functional mechanics of the disc, in other words, the transmission of weight load when the disc loses contact with the adjacent bone elements, since for the disc to suitably transmit the load, it is necessary to have precise contact with both endplates of the adjacent vertebrae, and in fractures with vertebral bone sinking, the contact either does not exist or is not uniform. Consequently, the normal sagittal curves of the spinal column are also altered, giving rise to kyphotic changes with negative biomechanical consequences for the patient.

Even in minor fractures, especially at critical levels such as the thoraco-lumbar transition or lower lumbar levels where mechanical and mobility stresses are greater, disc functionality is lost and compression of the neighboring neurological structures and very high mechanical stresses are produced on the ligamentous elements of the middle and posterior column that lead to their progressive degeneration. Consequently, it is of great importance to correct the anterior disc instability of the fractured level, recover disc functionality and maintain vertebral mobility of the injured segment percutaneously, as is done in this proposal.

The disc injury or injuries produced with the vertebral fracture will be treated percutaneously, correcting post-fracture kyphosis by replacing the disc with an elastic disc prosthesis that allows correcting and maintaining the correction of the vertebral deformity while maintaining the mobility of the fractured level.

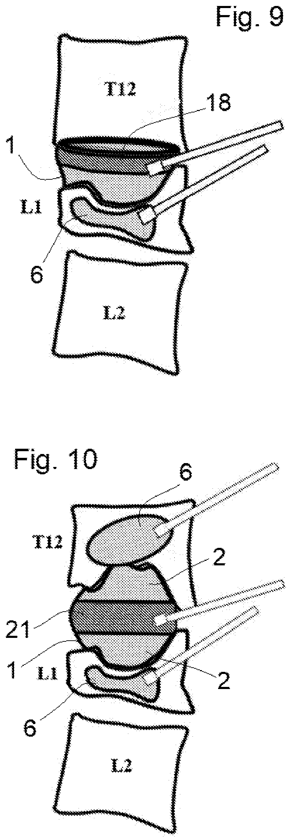

Figure 9 shows the repair of a severe fracture of the L1 (Kummel) vertebra, without affecting the T12 endplate, treated percutaneously and using implants such as those of the present invention. For this, a kyphoplasty of the L1 body is first performed with a balloon (1) to lift the height of the L1 vertebral body as much as possible. Next, the kyphotic deformity at this level is corrected and stabilized by inserting into the T12-L1 disc a preformed balloon (6) with a flat surface adapted to the T12 distal endplate, which is shaped as rigid or semi-rigid when filled with a bone cement. Inflating this balloon lifts the anterior spinal column and corrects the kyphotic vertebral deformity. Filling the balloon with elastic cement maintains the correction and allows vertebral mobility.

As previously mentioned, in this type of fracture two different balloons are used: a first balloon (6) that is placed inside the fractured vertebral body and is completely elastic, and a second balloon (1) that is inserted into the fractured space and also into the space where the disc was located before the fracture. This preformed balloon (1) has two compartments: a disc-shaped compartment (18), which adapts to the vertebral endplate that is not sunken, and another lower compartment that adapts to the sunken vertebral plate. The compartment (18) is cylindrical, since it represents the disc morphology and will be filled with a bone cement having the consistency and elasticity of the bone to be replaced, while the other compartment will be filled with an elastic cement (2) that is comparable to normal disc elasticity.

This balloon (1) is inflated after inserting the first balloon (6) inside the vertebral body to correct the height of that vertebra and fill it with the elastic material. Inflating this balloon (1) allows focal kyphosis at this level to be corrected under radioscopic control.

A fracture of the two vertebral plates adjacent to the disc (Figure 10) requires three balloons to be used to repair it:
Respective balloons (6), which are inserted into each of the two fractured vertebrae to correct, after inflation, the height of the vertebral bodies following the previously indicated methodology.

The third balloon (1) is inserted at the height of the injured disc. In the example of Figure 10, the third balloon (1) is divided into three compartments according to substantially horizontal planes, capable of being inflated independently. The intermediate compartment forms the intervertebral disc and is first filled with an elastic cement (21) having a consistency similar to that of the normal disc, and finally the two compartments, upper and lower, are filled with a cement (2) having elasticity greater than or equal to the cement (21) so that the balloon (1) adapts at the top and internally to the two irregular fractured surfaces of the adjacent vertebrae.

Once the nature of the invention has been described, as well as a preferred exemplary embodiment, it is clear that the invention is capable of industrial application, in the indicated sector.

It is also stated for appropriate purposes that the materials, shape, size and arrangement of the elements described may be modified, as long as this does not imply an alteration of the essential features of the invention that are claimed below:

## Claims

1. An implant for the intervertebral disc space for the treatment of scoliosis, kyphosis, stenosis and fractures of the spinal column, which comprises a substantially cylindrical, inflatable, flexible, biocompatible element that is suitable for insertion into the intervertebral disc space and can be inflated by an external pressure system in order to allow the injured disc to be lifted, wherein at least one of said bases (11) is rigid or semi-rigid and both bases (11) have a perimeter morphology dependent on the problem to be treated; while the perimeter envelope (12) is flexible and its height is determined by the separation that must exist between the intervertebral discs once said balloon is inflated; and wherein it is filled internally with a material (2) which, once solidified, has an elastic hardness similar to that of the healthy intervertebral disc.

2. The implant, according to claim 1, wherein the perimeter envelope (12), once inflated, acquires a height similar to that of the intervertebral disc, **characterized in that** both bases (11) are semi-rigid and their morphology corresponds to an irregular section in which at least one area (15) has the shape of a circular sector, which has a larger contact surface in the area of the annulus fibrosus (5) that must be lifted to repair vertebral deformities such as degenerative scoliosis, kyphosis, anterior vertebral imbalances and spondylolisthesis.

3. The implant, according to claim 1, **characterized in that** both bases (11) are semi-rigid and the perimeter envelope (12) has a central cylinder (16) and another peripheral cylinder (1), both independently inflatable and fillable, with a cement (2) having equal or less elasticity in the peripheral cylinder (1) than the cement (21) that fills the central cylinder (16), when it is to be used in cases of disc degeneration or lumbar spinal stenosis, in order to increase the stability of the implant and provide greater disc mobility, causing a smoother transition to the annulus fibrosus.

4. The implant, according to claim 1, **characterized in that** it comprises a preformed balloon (1) divided into two compartments: a disc-shaped compartment (18), which adapts to the vertebral endplate that is not sunken, which will be filled with elastic cement having the consistency and elasticity of the disc to be replaced, forming a semi-rigid base, and a second compartment that will be filled with a bone cement (2) that is comparable to the elasticity of the adjacent bone, and which adapts to the irregular surface of a fractured vertebra, in which a second balloon (6) has been previously inserted into the vertebral body to correct the height of said vertebra, and an elastic material inserted therein which allows focal kyphosis at this level to be corrected, in the case of fracture of one of the vertebral plates adjacent to the disc.

5. The implant, according to claim 1, **characterized in that** it comprises a preformed balloon (1) to be inserted into the injured disc, said balloon being divided into three compartments according to substantially horizontal planes, capable of being inflated independently, wherein the intermediate compartment forms the intervertebral disc and is first filled with an elastic cement (21) having a consistency similar to that of the normal disc, while the two compartments, upper and lower, are filled with a cement (2) having greater or equal elasticity similar to that of the adjacent bone (21) so that the balloon (1) adapts at the top and internally to the two irregular fractured surfaces of the adjacent vertebrae, in which the respective balloons (6) have been previously placed to correct, after inflation, the height of the vertebral bodies.

6. The implant, according to any of the preceding claims, **characterized in that** it has radiopaque markers (17) on both bases that allow the exact location of the implant to be observed when it is placed between the two vertebrae.

7. The implant, according to any of the preceding claims, **characterized in that** the elastic hardness of the filling material (2) of the balloon (1) is comprised between Shore 50A and Shore 90A.

8. The implant, according to any of the preceding claims, **characterized in that** the material that fills the implant is a silicone, rubber, cement or any other elastic material, initially liquid or in the form of a gel, and that solidifies once injected inside of the balloon (1) acquiring the elastic hardness similar to that of the healthy intervertebral disc at the vertebral level in which the implant is to be inserted.

9. Use of a balloon in the intervertebral disc space comprising a substantially cylindrical, inflatable, flexible, biocompatible element suitable for insertion into the intervertebral disc space and can be inflated by an external pressure system in order to allow the injured disc to be lifted, wherein the bases (11) are rigid or semi-rigid and have an irregular perimeter section in which at least one area (15) has the shape of a circular sector, which has a larger contact surface in the area of the annulus fibrosus (5) that must be lifted, in order to repair vertebral deformities such as degenerative scoliosis, kyphosis, anterior vertebral imbalances and spondylolisthesis.

10. Use of a balloon in the intervertebral disc space comprising a substantially cylindrical, inflatable, flexible, biocompatible element suitable for insertion into the intervertebral disc space and can be inflated by an external pressure system in order to allow the injured disc to be lifted, wherein both bases (11) are semi-rigid and the perimeter envelope (12) has a central cylinder (16) and another peripheral cylinder (1), both independently inflatable and fillable, with cements having less or equal elasticity in the peripheral cylinder (1) than in the central cylinder (16), in cases of disc degeneration or lumbar spinal stenosis, in order to increase the stability of the implant and provide greater disc mobility, causing a smoother transition to the annulus fibrosus.

11. Use of a balloon in the intervertebral disc space comprising a substantially cylindrical, inflatable, flexible, biocompatible element suitable for insertion into the intervertebral disc space and can be inflated by an external pressure system in order to allow the injured disc to be lifted, said balloon being internally divided into two compartments: a disc-shaped compartment (18), which adapts to the vertebral endplate that is not sunken, which will be filled with bone cement having the consistency and elasticity of the bone to be replaced, and a second compartment that will be filled with an elastic cement (2) that is comparable to normal disc elasticity, which adapts to the irregular surface of a fractured vertebra, in which a second balloon (6) has been previously inserted into the vertebral body to correct the height of said vertebra, and an elastic material is inserted therein, in order to repair the fracture of one of the vertebral plates adjacent to the disc of a fractured vertebra.

12. Use of a balloon in the intervertebral disc space comprising a substantially cylindrical, inflatable, flexible, biocompatible element suitable for insertion into the intervertebral disc space and can be inflated by an external pressure system in order to allow the injured disc to be lifted, said balloon being internally divided into three sections according to substantially horizontal planes, capable of being inflated independently, the intermediate section forming a disc that is first filled with an elastic cement (21) having elasticity greater than or equal to the cement (2) that subsequently fills the two upper and lower sections are subsequently filled, in order to recover the vertebral height lost after the fracture of the adjacent vertebrae, in which respective balloons (6) have been previously placed to correct, after inflation, the height of the vertebral bodies.
